Europäisches Patentamt

European Patent Office

Office européen des brevets

(11) Publication number: **0 048 402**
**B1**

# (12) EUROPEAN PATENT SPECIFICATION

(45) Date of publication of patent specification: **14.08.85**

(51) Int. Cl.⁴: **A 61 N 5/02,** A 61 B 1/00

(21) Application number: **81107120.8**

(22) Date of filing: **10.09.81**

(54) Endoscope apparatus.

(30) Priority: **18.09.80 JP 129480/80**

(43) Date of publication of application:
**31.03.82 Bulletin 82/13**

(45) Publication of the grant of the patent:
**14.08.85 Bulletin 85/33**

(84) Designated Contracting States:
**AT BE CH DE FR GB IT LI NL SE**

(56) References cited:
EP-A-0 044 538
CH-A- 254 655
DE-A-1 648 905
DE-A-3 012 150
FR-A-2 269 356
FR-A-2 421 628
GB-A-2 000 335
US-A-3 924 196

PROCEEDINGS OF THE IEEE, vol. 68, no. 1,
January 1980, New Jersey, US, L.S. TAYLOR
"Implantable radiators for cancer therapy by
microwave hyperthermia", pages 142-149

(73) Proprietor: **OLYMPUS OPTICAL CO., LTD.**
**43-2, 2-chome, Hatagaya Shibuya-ku**
**Tokyo 151 (JP)**

(72) Inventor: **Miyamoto, Yoshihiko**
**7-17-16, Oowada-machi Hachioji-shi**
**Tokyo (JP)**

(74) Representative: **Gramm, Werner, Prof. Dipl.-Ing.**
**et al**
**Patentanwälte Gramm + Lins Theodor-Heuss-**
**Strasse 2**
**D-3300 Braunschweig (DE)**

(56) References cited:
**IEEE TRANSACTIONS ON INSTRUMENTATION**
**AND MEASUREMENT, vol. IM-29, no. 2, June**
**1980, New York, US, D.D. NGUYEN et al.**
**"Combination of local heating and radiometry**
**by microwaves", pages 143-144**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Description

The present invention relates to an endoscope apparatus for detecting and treating affected parts in a body cavity, using microwaves.

A type of medical therapy is already known according to which an affected part such as a malignant tumor in a living body is heated to destroy the cells in this part. However, no effective means according to this therapy has been established yet. For example, a method for heating and treating the affected part in the body cavity is known. This method utilizes an air and water supply inlet and a suction outlet provided at the distal end portion of the endoscope. Warm air or water is blown on the affected part in the body cavity from this air and water supply inlet, while the warm air or water is exhausted from the suction outlet, in order to heat and treat the affected part. This method provides some therapy effect when the malignant tumor portion is exposed at the surface of the body. However, when the tumor lies deep in the body, the warm air or water blown against the vital tissues will stimulate the circulation of blood in the normal tissue at the surface of the cavity wall. The temperature rise in the tissues is thus suppressed and heat cannot be transferred to the deep portion, so that the therapy effect at the deep part may not be obtained.

Another method employs conventional endoscopic high frequency surgical knives and endoscopic laser scalpels. But these instruments are essentially used to surgically remove the affected parts, so that accidental contact of such an instrument with normal tissue may destroy this tissue. If the high frequency output or the laser output is reduced to avoid such a danger, the self-protection function of the normal tissue described above prevents the energy from being transferred to the deep affected part, and thus a sufficient treatment effect is not obtained.

On the other hand, when the affected part in the body cavity is to be treated, the presence of the affected part and its position must be detected in advance. However, it has been conventionally impossible to continuously carry out detection and treatment of the affected part utilizing the various means described above. After the detecting means is inserted into the body cavity through a channel of the endoscope to detect the affected part, it is extracted from the channel and then a treating means such as a high frequency or laser scalpel is inserted through this channel to treat the affected part. Therefore, much time and labor is required for the detection and the treatment of the affected part, which results in poor operability. In addition, since the endoscope may shift in the body cavity when the affected part detected by the detecting means is treated by the treating means, the affected part cannot be reliably treated.

The afore-mentioned detecting means utilizes for detection of the affected part the fact that the temperature of the affected part such as cancer or a tumor in the vital tissues is higher by about 1°C than that of normal tissue. For example, a probe for measuring temperature is inserted into the channel and then pressed against the inner surface of the body cavity to measure the temperature thereof, or a liquid crystal membrane is pressed against the inner surface of the body cavity and the variation in color of this membrane is observed to determine the temperature.

However, since these detecting means utilize heat conduction through contact, only the temperature at the surface of the body can be measured and therefore an affected part lying below the mucous membranes cannot be precisely detected. It is, of course, entirely impossible to find an affected part in the pancreas or the liver into which the detecting means cannot be inserted.

In summary, it has been conventionally difficult to detect and treat an affected part below the surface of the body cavity or to continuously perform these detection and treatment operations easily and rapidly.

The content of EP—A—0 044 538 (priority date; 23.07.80, publication date: 27.01.82) according to Article 54 (3) and (4) is considered to be comprised in the state of the art.

In this document there is proposed an endoscope apparatus including an endoscope having an insertion tube with a distal end portion comprising microwave means for transmitting microwaves and an objective lens system, said microwave means comprising a microwave antenna comprised by an antenna rod, a reflector and a protective membrane, for radiating treatment microwaves to the vital tissue.

The articles in Proc. IEEE, Volume 68, No. 1, January 1980, "Implantable Radiators for Cancer Therapy by Microwave Hyperthermia", L. S Taylor, pp. 142—149, proposes to combine an endoscope and a microwave radiator.

The patent application FR—A—2 421 628 describes an implantable electrode which radiates microwaves and receives microwaves for the determination of the temperature of the surrounding tissue.

It is an object of this invention to provide an endoscope apparatus for rapidly and continuously carrying out detection and treatment of an affected part in a body cavity.

According to this invention, the distal end portion of the endoscope is provided with a microwave antenna for detecting microwaves radiated from the vital tissues and for radiating microwaves to the vital tissues, wherein said distal end portion includes an objective lens system, the lens system and microwave means being arranged on the front end of the insertion tube with their axes parallel to that of the tube; said microwave means includes a microwave antenna comprised by an antenna rod, a reflector for providing directivity in the general direction of the field of view of the objective lens system, and a protective membrane fitted over the front opening of said reflector, said microwave antenna being provided at said distal end portion for receiving microwaves radiated from vital tissues in the body cavity and for radiating treatment microwaves to the vital tissue; and that said endoscope apparatus further includes receiving means provided for converting the biological radiant microwave to a reception signal corresponding to the intensity of the biological radiant microwave.

The affected part is detected based on the level of intensity of the microwaves radiated from the vital tissues, and is treated by microwaves radiated on the detected affected part.

This invention can be more fully understood from the following detailed description when taken in conjunction with the accompanying drawings, in which:

Fig. 1 shows a perspective view of an endoscope apparatus according to one embodiment of this invention;

Fig. 2 is a view showing the internal structure of the endoscope apparatus shown in Fig. 1;

Fig. 3 is a sectional view of the distal end portion of the same endoscope;

Fig. 4 is a sectional view of the distal end portion of an endoscope apparatus according to another embodiment of this invention; and

Fig. 5 is a view showing the general structure of the endoscope apparatus according to the embodiment of Fig. 4.

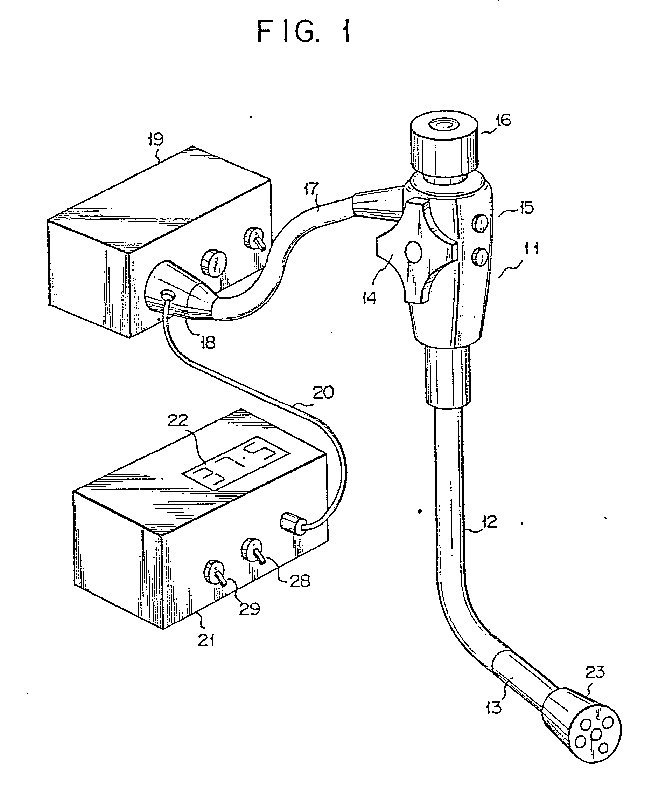

Referring to an endoscope apparatus as shown in Fig. 1, an endoscope 11 comprises an insertion tube 12 which is inserted into a body cavity, a control section 15 having an angle knob 14 for bending a bending section 13 of this insertion tube 12, and an eyepiece section 16 for observing the interior of the body cavity. A connector 18 provided at the front end of a universal cord 17 extending from the control section 15 of the endoscope 11 is coupled to a socket (not shown) of a light supply unit 19. To the connector 18 is also connected a microwave transmitting and receiving device 21 (hereinafter referred to as the "microwave device") via a transmission line for passing a microwave current therethrough, for example, a coaxial cable 20. A display 22 is provided at this microwave device 21.

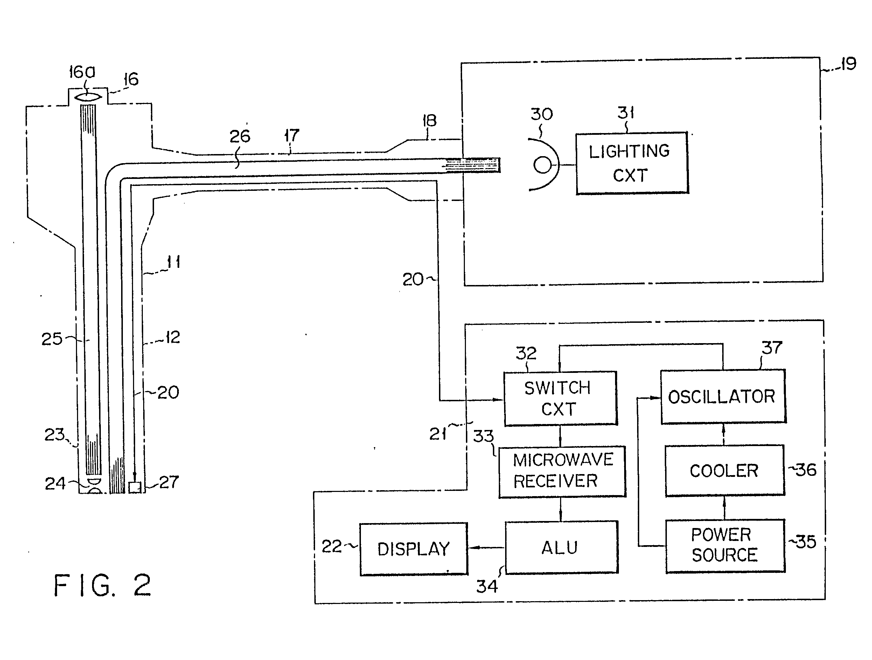

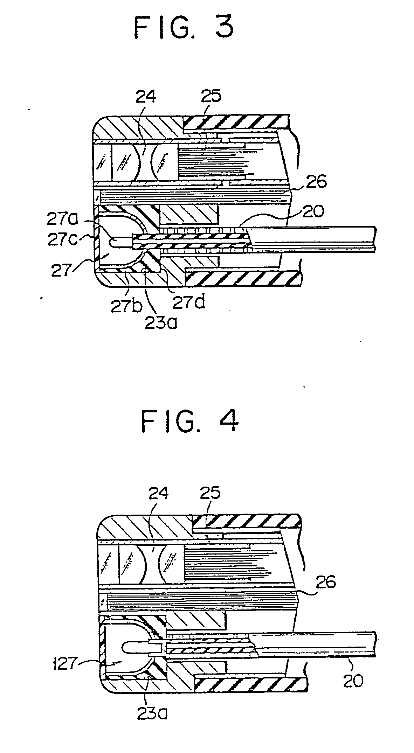

The schematic internal structure of the endoscope apparatus of Fig. 1 is shown in Fig. 2. As shown in this figure, an objective lens system 24 is provided at the distal end 23 of the endoscope 11, and an optical image guide 25 extends from the objective lens system 24 to an eyepiece lens 16a of the eyepiece section 16. A light guide 26 extends from the distal end 23 to the insertion tube 12 and through the universal cord 17 to the connector 18. The distal end 23 is provided with a microwave antenna 27 which is connected to the microwave transmission line (coaxial cable) 20. Referring now to Fig. 3 showing the structure of the distal end 23 in detail, the microwave antenna 27 is composed of an antenna rod 27a and a microwave reflector 27b surrounding the antenna rod 27a. The reflector 27b has a reflective inner surface 23a of parabolic shape and is made of a metallic material for prevention of heat radiation. Over the front opening of the reflector 27b is liquid-tightly fitted a protective membrane 27c which is made of a material having a low dielectric constant, for example, vinyl chloride. The outer surface of the reflector 27b is covered with a cover 27d for preventing heat radiation and for insulation. This cover 27d is made of a high-heat-resistant insulating material of high strength, for example, epoxy resin, ceramic or the like. The microwave antenna 27 thus constructed is fitted into and fixed in a recess 23a provided in the distal end 23. The cover 27d is not required if the distal end 23 is made of the same material as that of the cover 27d. The microwave antenna 27 is connected to a core 20a of the coaxial cable 20. The light guide 26 is disposed adjacent to the microwave antenna 27, and the objective lens system 24 and the image guide 25 are juxtaposed to the light guide 26.

When the connector 18 is coupled to the light supply unit 19, the light guide 28 guides the light from a light source 30 in the light supply unit 19 into the body cavity. The light source 30 is operated and controlled by a lighting circuit 31. The microwave transmission line, i.e., the coaxial cable 20, is connected to one input terminal of a switch circuit 32 of the microwave device 21. The output terminal of this switch circuit 32 is connected to the input terminal of a microwave receiver 33, the output terminals of which is connected to the input terminal of an arithmetic circuit 34. The output terminal of the arithmetic circuit 34 is connected to the display 22. A power source 35 is connected to a cooler 36 and an oscillator 37 to supply power thereto. The cooler 36 is disposed so as to cool an oscillating tube (not shown) of the oscillator 37. The output terminal of the oscillator 37 is connected to the other output terminal of the switch circuit 32.

In the endoscope apparatus of this structure, when a switch 28 of the microwave device 21 is changed over to the reception side and the insertion tube 12 of the endoscope 11 is inserted into the body cavity, microwaves generating from the tissues lying in the direction of the observation field of view of the eyepiece section 16 are received by the microwave antenna 27 at the distal end 23. These microwaves are input to the switch circuit 32 through the coaxial cable 20. The switch circuit 32 guides the microwaves as a microwave current to the microwave receiver 33, since the switch 28 has been changed over to the reception side. The microwave receiver 33 shapes the waveform of the microwave current, amplifies it, and then inputs it into the arithmetic circuit 34. The arithmetic circuit 34 calculates the temperature corresponding to the intensity of the microwaves i.e. the temperature of the vital tissues, on the basis of the microwave current. The temperature data obtained from this calculation is input to the display 22 for display. The affected part such as cancer or a tumor in the vital tissues has a temperature higher by 1°C than that of normal tissue. Therefore, when the high temperature is displayed on the display 22, the existence of an affected part in the direction of the field of view can be detected. When the affected part is detected, the switch 28 is changed over to the transmission side. Then, a microwave current in the oscillator 37 is guided into the coaxial cable 20 via the switch circuit 32. The microwave current in the coaxial cable 20 is supplied to the microwave antenna 27, and then microwaves are radiated from the microwave antenna 27 to the affected part. If the affected part lies deep beneath the wall of the body cavity, the affected part is irradiated with the microwaves through the normal tissue at the cavity wall surface. In this case, the temperature of the normal tissues is suppressed due to the simulated blood circulation caused by the self-protective function of the body. However, the temperature in the affected part rises in correspondence with the microwave intensity, since the affected part has bad blood circulation. When this raised temperature reaches a predetermined value, the tissues of the affected part are destroyed by the heat and the affected part may be treated.

As described above, according to this invention, microwaves are used for detection and therapy of the affected part of the vital tissues in the body cavity. Microwaves are capable of being transmitted through vital tissues. Therefore, even if the affected part lies deep beneath the cavity wall, the microwaves radiated from the affected part penetrate through the normal tissues at the cavity wall surface and are received by the microwave antenna. In this manner, the deep affected part can be easily detected. Similarly, microwaves are also transmitted through the normal tissues and arrive at the deep affected part in the case of therapy. In the detection and treatment of the affected part, reception and transmission of the microwaves can be executed by merely changing over the switch, so that the apparatus has excellent operability and assures accurate radiation of the microwaves to the affected part after detection.

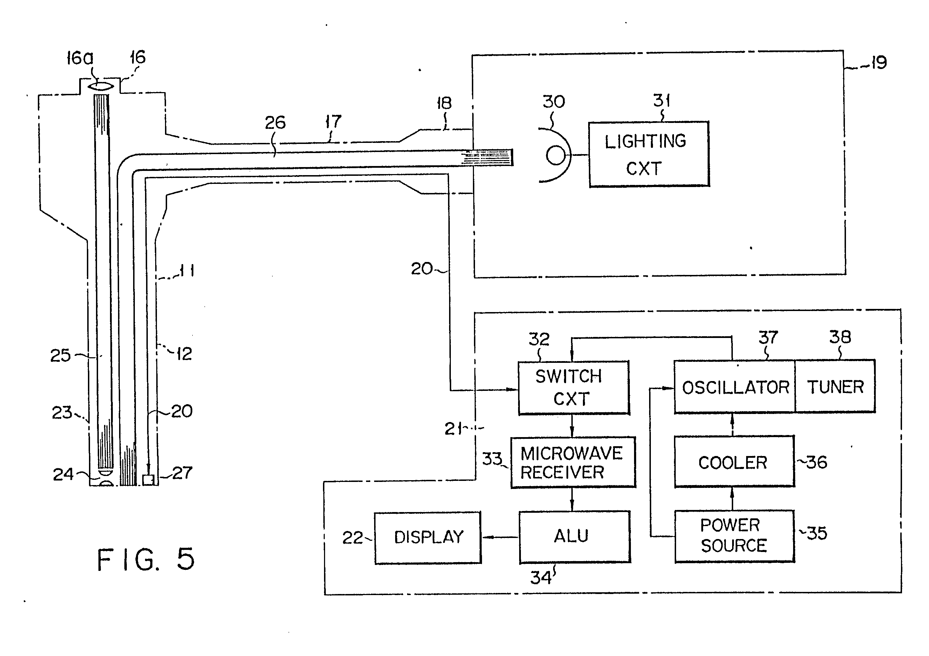

In the embodiment described above, the microwave antenna has been fixed to the distal end of the endoscope. However, it may be detachably provided at the distal end as shown in Fig. 4. In other words, the microwave antenna 27 is detachably screwed or snapped in the recess 23a of the distal end 23. When the antenna 27 is mounted in the recess 23a, it is connected to the coaxial cable 20. With such a construction, the antenna may be replaced by another of a given frequency band, so that microwaves may be received with high characteristics. In addition, the oscillator 37 in the microwave device 21 is provided with a tuner 38 as shown in Fig. 5, and the microwaves oscillated from the oscillator 37 are adjusted in frequency.

In accordance with this structure, the frequency of the microwaves which can be received or oscillated can be varied according to the conditions of the affected part so that good detection and treatment thereof may be achieved.

Although a pole antenna is employed as the antenna 27 in the above embodiment, a dipole antenna may be used. Additionally, although the temperature was displayed by the display provided in the microwave device, the display may be provided in the field of view of the endoscope eyepiece section for performing temperature display. The display does not necessarily display the temperature; it is sufficient if the indication helps to differentiate the affected part from normal tissue.

## Claims

1. An endoscope apparatus including an endoscope having an insertion tube for insertion into a body cavity, a distal end portion provided at a front end of said insertion tube, and microwave means for receiving and transmitting microwaves, wherein said distal end portion includes an objective lens system, the lens system and microwave means being arranged on the front end of the insertion tube with their axes parallel to that of the tube; said microwave means includes a microwave antenna (27) comprised by an antenna rod (27a), a reflector (27b) for providing directivity in the general direction of the field of view of the objective lens system, and a protective membrane (27c) fitted over the front opening of said reflector (27b), said microwave antenna (27) being provided at said distal end portion for receiving microwaves radiated from vital tissues in the body cavity and for radiating treatment microwaves to the vital tissue; and that said endoscope apparatus further includes receiving means (33) provided for converting the biological radiant microwave to a reception signal corresponding to the intensity of the biological radiant microwave.

2. An endoscope apparatus according to claim 1, characterized in that said microwave antenna (27) is detachably mounted to said distal end portion (23).

3. An endoscope apparatus according to claim 1, characterized in that a coaxial cable (20) is connected to said microwave antenna (27) for transmitting the microwave.

4. An endoscope according to claim 1, 2 or 3, characterized in that the biological radiant microwave and treatment microwave are selectively changed over by switching means (32).

5. An endoscope apparatus according to claim 1, characterized in that said reception signal from said receiving means (33) is input into arithmetic means (34) for calculating and processing said reception signal to compute a temperature of the vital tissues corresponding to the microwave intensity and for inputting temperature data to said displaying means (22) for display.

6. An endoscope apparatus according to claim 1, characterized in that said protective membrane (27c) is made of a material having a low dielectric constant.

7. An endoscope apparatus according to claim 1, characterized in that said protective membrane (27c) is made of vinyl chloride.

8. An endoscope apparatus according to claim 1, characterized in that said microwave antenna (27) has a cover (27d) provided on the outer surface of said reflector (27b), said cover being made of a highly heat resistant insulating material of high strength.

9. An endoscope apparatus according to claim 8, characterized in that said cover (27d) is made of an epoxy resin.

10. An endoscope apparatus according to claim 8, characterized in that said cover (27d) is made of ceramic.

## Patentansprüche

1. Ein endoskopisches Gerät mit einem Endoskop mit einem Einführungsrohr zur Einführung in eine Körperhöhle, einem distalen Ende am vorderen Ende des Einführungsrohres und einer Mikrowelleneinrichtung zum Empfang und Aussenden von Mikrowellen, wobei das distale Ende ein Objektivlinssystem aufweist, das zusammen mit der Mikrowelleneinrichtung am vorderen Ende des Einführungsrohres angebracht ist und deren Achsen parallel zu der Achse des Rohres liegen, wobei die Mikrowelleneinrichtung eine Mikrowellenantenne (27) mit einem Antennenstab (27a), einem Reflektor (27b) zur Ausrichtung in die allgemeine Richtung des Blickfeldes des Objektivlinssystems und einer an der vorderen Öffnung des Reflektors (27b) angebrachten Schutzmembrane (27c) aufweist, wobei die Mikrowellenantenne (27) am distalen Ende angebracht ist, um die von Lebendgeweben in der Körperhöhle ausgestrahlten Mikrowellen zu empfangen und die Behandlungsmikrowellen in die Lebendgewebe auszustrahlen, und wobei das endoskopische Gerät Empfangseinrichtungen (33) aufweist zur Umwandlung der biologischen Mikrowelle in ein der Intensität der biologischen Mikrowelle entsprechendes Empfangssignal.

2. Endoskopisches Gerät nach Anspruch 1, dadurch gekennzeichnet, daß die Mikrowellenantenne (27) lösbar an dem distalen Ende (23) montiert ist.

3. Endoskopisches Gerät nach Anspruch 1, dadurch gekennzeichnet, daß ein koaxiales Kabel (20) mit der Mikrowellenantenne (27) zur Übertragung der Mikrowelle verbunden ist.

4. Endoskopisches Gerät nach Anspruch 1, 2 oder 3, dadurch gekennzeichnet, daß es wahlweise auf die biologische Mikrowelle und die Behandlungsmikrowelle durch Schaltvorrichtungen (32) umgeschaltet wird.

5. Endoskopisches Gerät nach Anspruch 1, dadurch gekennzeichnet, daß das Empfangssignal von der Empfangseinrichtung (33) zur Berechnung und Bearbeitung des Empfangssignals, um eine Temperatur der Lebendgewebe entsprechend der Mikrowellenintensität zur berechnen, und zur Eingabe der Temperaturdaten in das Sichtanzeigegerät (22) zur Anzeige in eine Rechenstufe (34) eingegeben wird.

6. Endoskopisches Gerät nach Anspruch 1, dadurch gekennzeichnet, daß die Schutzmembrane (27c) aus einem Material mit einer niedrigen dielektrischen Konstanten besteht.

7. Endoskopisches Gerät nach Anspruch 1, dadurch gekennzeichnet, daß die Schutzmembrane (27c) aus Vinylchlorid besteht.

8. Endoskopisches Gerät nach Anspruch 1, dadurch gekennzeichnet, daß die Mikrowellenantenne (27) an der äußeren Oberfläche des Reflektors (27b) eine aus hochhitzbeständigem, hochfestem Isoliermaterial bestehende Hülle (27d) aufweist.

9. Endoskopisches Gerät nach Anspruch 8, dadurch gekennzeichnet, daß die Hülle (27d) aus Epoxidharz besteht.

10. Endoskopisches Gerät nach Anspruch 8, dadurch gekennzeichnet, daß die Hülle (27d) aus Keramik besteht.

## Revendications

1. Appareil endoscopique comprenant un endoscope comportant un tube d'introduction à introduire dans une cavité du corps, une partie d'extrémité distale prévue à une extrémité antérieure du tube d'introduction, et un dispositif à micro-ondes pour recevoir et émettre des micro-ondes, la partie d'extrémité distale comprenant un système d'objectif, le système d'objectif et le dispositif à micro-ondes étant disposés à l'extrémité antérieure du tube d'insertion, de telle façon que leurs axes soient parallèles à celui du tube, le dispostif à micro-ondes comprenant une antenne à micro-ondes (27) formée d'un barreau d'antenne (27a), d'un réflecteur (27b) conférant de la directivité à la direction générale du champ d'observation du système d'objectif, et d'une membrane protectrice (27c) montée sur l'orifice antérieur du réflecteur (27b), l'antenne à micro-ondes (27) étant prévue à la dite partie d'extrémité distale pour recevoir des micro-ondes émises par des tissus vitaux dans la cavité du corps et pour émettre des micro-ondes de traitement en direction des tissus vitaux et l'appareil endoscopique comprenant, en outre, un dispositif récepteur (33) prévu pour convertir les micro-ondes de rayonnement biologique en un signal de réception correspondant à l'intensité des micro-ondes de rayonnement biologique.

2. Appareil endoscopique suivant la revendication 1, caractérisé en ce que l'antenne à micro-ondes (27) est montée de façon détachable sur la partie d'extrémité distale (23).

3. Appareil endoscopique suivant la revendication 1, caractérisé en ce qu'un câble coaxial (20) est connecté à l'antenne à micro-ondes (27) pour transmettre les micro-ondes.

4. Endoscope suivant la revendication 1, 2 ou 3, caractérisé en ce que les micro-ondes de rayonnement biologique et les micro-ondes de traitement sont permutées sélectivement par un dispositif de commutation (32).

5. Appareil endoscopique suivant la revendication 1, caractérisé en ce que le signal de réception du dispositif récepteur (33) est introduit dans un dispositif arithmétique (34) pour calculer et traiter le signal de réception en vue de déterminer une température des tissus vitaux correspondant à l'intensité des micro-ondes et pour introduire des données de température dans le dispositif d'affichage (22) en vue de leur présentation.

6. Appareil endoscopique suivant la revendication 1, caractérisé en ce que la membrane protectrice (27c) est faite d'une matière présentant une constante diélectrique peu élevée.

7. Appareil endoscopique suivant la revendication 1, caractérisé en ce que la membrane protectrice (27c) est en chlorure de vinyle.

8. Appareil endoscopique suivant la revendication 1, caractérisé en ce que l'antenne à micro-ondes (27) comporte une coiffe (27d) prévue sur la surface externe du réflecteur (27b), cette coiffe étant faite d'une matière isolante à haute résistance mécanique et thermique.

9. Appareil endoscopique suivant la revendication 8, caractérisé en ce que la coiffe (27d) est faite d'une résine époxyde.

10. Appareil endoscopique suivant la revendication 8, caractérisé en ce que la coiffe (27d) est en céramique.

# FIG. 1

FIG. 2

0 048 402

**0 048 402**

# FIG. 3

# FIG. 4

FIG. 5

0 048 402